# EUROPEAN PATENT SPECIFICATION

(11) **EP 2 513 099 B1**
(45) Date of publication and mention of the grant of the patent: **20.11.2019**
(21) Application number: 10794975.2
(22) Date of filing: 15.12.2010
(51) Int. Cl.: C07D 451/02

(54) **PREPARATION OF N-MONOFLUOROALKYL TROPANES**
HERSTELLUNG VON MONOFLUORALKYLTROPANEN
PRÉPARATION DE TROPANES N-MONOFLUOROALKYLÉS

(30) Priority: 17.12.2009 GB 0922023; 17.12.2009 US 287264 P
(43) Date of publication of application: 24.10.2012
(73) Proprietor: GE Healthcare Limited, Little Chalfont, Buckinghamshire HP7 9NA (GB)
(72) Inventor: WILLIAMS, Lorenzo, N-4220 Nydalen Oslo (NO); KEILEN, Gunnar, N-4220 Nydalen Oslo (NO); HAUGAN, Jarle Andre, N-4220 Nydalen Oslo (NO)
(74) Representative: Bannan, Sally
(86) International application number: PCT/EP2010/069758
(87) International publication number: WO 2011/073256

(56) References cited:
- WO-A1-2006/065038
- NEUMEYER J L ET AL: "N-OMEGA-FLUOROALKYL ANALOGS OF (IR)-2BETA-CARBOMETHOXY-3BETA-(4-IODOP HENYL)-TROPANE NBETA-CIT): RADIOTRACERS FOR POSITRON EMISSION TOMOGRAPHY AND SINGLE PHOTON EMISSION COMPUTED TOMOGRAPHY IMGAGING OF DOPAMINE TRANSPORTERS", JOURNAL OF MEDICINAL CHEMISTRY, AMERICAN CHEMICAL SOCIETY, WASHINGTON, US, vol. 37, no. 11, 1 January 1994 (1994-01-01), pages 1558-1561, XP001053024, ISSN: 0022-2623, DOI: DOI:10.1021/JM00037A004 cited in the application
- KLOK R P ET AL: "Synthesis of N-(3-[18F]fluoropropyl)-2.beta.-carbo- methoxy-3.beta.-(4-iodophenyl)nortropane ([18F]FP-.beta.-CIT)", JOURNAL OF LABELLED COMPOUNDS AND RADIOPHARMACEUTICALS, JOHN WILEY, CHICHESTER, GB, vol. 49, no. 2, 10 February 2006 (2006-02-10), pages 77-89, XP002501239, ISSN: 0362-4803, DOI: DOI:10.1002/JLCR.1043
- "XIIth international symposium on radiopharmaceutical chemistry: Abstracts and programme", Journal of Labelled Compounds and Radiopharmaceuticals, vol. 40, no. 8, 1 August 1997 (1997-08-01) , pages 515-594, XP55056991, ISSN: 0362-4803, DOI: 10.1002/jlcr.2580400801
- Satendra Singh: "Chemistry, Design, and Structure-Activity Relationship of Cocaine Antagonists", Chemical Reviews, vol. 100, no. 3, 1 March 2000 (2000-03-01) , pages 925-1024, XP055133125, ISSN: 0009-2665, DOI: 10.1021/cr9700538

## Description

### Field of the Invention.

The present invention relates to an improved synthesis of *N*-monofluoroalkyl tropanes using fluoroalkyl iodides or fluoroalkyl sulfonate esters. The invention also provides the use of such method to prepare the non-radioactive tropane intermediate FP-CIT, and its subsequent conversion to the ¹²³I-labelled radiopharmaceutical DaTSCAN™ (¹²³I-ioflupane). Also provided is the use of fluoroalkyl iodides or fluoroalkyl sulfonate esters in the alkylation method of the invention.

### Background to the Invention.

DaTSCAN™ (¹²³I-ioflupane or ¹²³I FP-CIT) is prepared as follows [Neumeyer et al, J.Med.Chem., 37, 1558-1561 (1994)]:

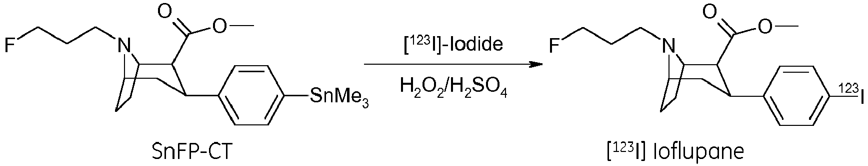

The trialkyltin non-radioactive precursor SnFP-CT is prepared from nor-beta-CIT as follows [Neumeyer et al, J.Med.Chem., 37, 1558-1561 (1994)]:

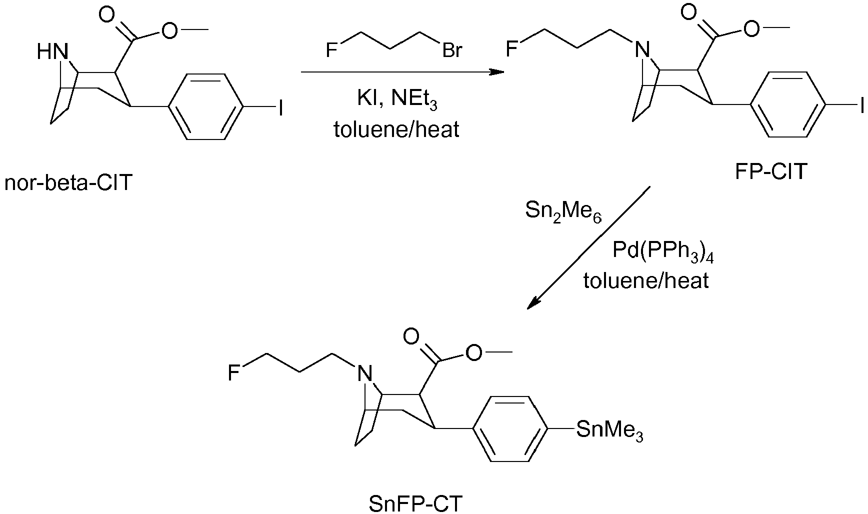

Chi et al [J. Org. Chem., 52, 658-664 (1987)] describe a method for the *N-*fluoroalkylation of amides and amines. The chemistry described is non-radioactive, but is designed to be suitable for adaptation to the synthesis of the corresponding ¹⁸F-labelled analogues. Spiperone was used as the model amide for amide *N*-alkylation, and phenylpiperazine as the model amine for amine *N*-alkylation. The route uses the steps:
(i) fluoride ion displacement of a haloalkyl triflate (i.e. trifluoromethanesulfonate) to give a fluoroalkyl halide;
(ii) *N*-alkylation of an amide/amine by the fluoroalkyl halide from step (i).

Chi *et al* use only fluoroalkyl bromides for amine *N*-alkylation.

Shiue et al [J.Lab.Comp.Radiopharm., 24, 55-64 (1987)] describe the synthesis of [¹⁸F]-fluoroalkylhalides as follows:

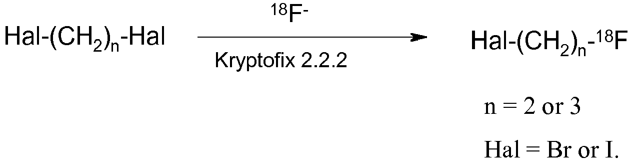

The [¹⁸F]-fluoroalkylhalides prepared were used to *N*-alkylate spiroperidol, normetazocine and lorazepam.

Teng et al [Nucl. Med. Biol., 17(8), 811-817 (1990)] describe the synthesis of the SCH analogues shown:

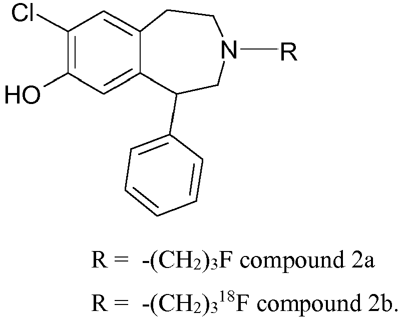

Compound 2a of Teng is the non-radioactive (¹⁹F) derivative, and is prepared by *N-*alkylation of the R = H compound with 1-bromo-3-fluoropropane. Compound 2b of Teng is the radioactive (¹⁸F) derivative, and is prepared by *N*-alkylation of the R = H compound with 3-[¹⁸F]fluoro-1-iodo-propane.

Lannoye et al [J.Med.Chem., 33, 2430-2437 (1990)] prepare *N*-fluoroalkyl analogues of the dopamine D-2 receptor antagonist raclopride. The alkylating agents used were 1-bromo-3-fluoropropane and 1-bromo-2-fluoroethane. Halldin et al [Nucl.Med.Biol., 18(8), 871-881 (1991)] disclose the preparation of *N*-fluoroalkyl salicylamides, such as raclopride and eticlopride, having N-(CH₂)ₙ^{y}F substituents, where n is 2 or 3 and y is 18 or 19. The synthesis uses *N*-alkylation of the secondary amine substituent with the bromo-fluoroalkane Br-(CH₂)ₙ^{y}F.

Swahn et al [J.Lab.Comp.Radiopharm., 38, 675-685 (1996)] describe the synthesis of FP-CIT, and the corresponding N-(2-fluoroethyl) analogue (CIT-FE) *via N*-alkylation of nor-beta-CIT using 1-bromo-3-fluoropropane and 1-bromo-2-fluoroethane respectively.

Lundkvist et al [Nucl.Med.Biol., 24, 621-627 (1997)] prepare the ¹⁸F-labelled analogue of ¹²³I-ioflupane, where the ¹⁸F radiolabel is located in the *N*-fluoropropyl group, *via N*-alkylation of nor-beta-CIT using ¹⁸F-(CH₂)₃-Br.

Stehouwer et al [J.Med.Chem., 48, 7080-7083 (2005)] prepare *N*-fluoroalkyl furan-substituted tropanes as follows:

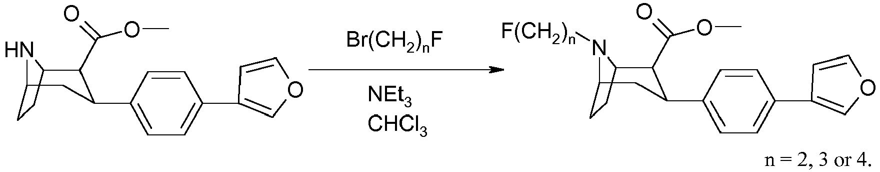

Yu et al [Bioorg.Med.Chem., 16, 6145-6155 (2008)] prepare *N*-fluoroalkyl isoquinoline carboxamide derivatives, *via N*-alkylation of a secondary amide using sodium hydride and 1-bromo-3-fluoropropane or 1-bromo-2-fluoroethane.

Export or import of 1-bromo-3-fluoropropane (BFP) and related chemicals are, however, now prohibited worldwide due to their ozone-depleting properties. There is therefore a need for alternative viable syntheses of drugs and imaging agents which comprise *N*-fluoroalkyl substituents.

### The Present Invention.

The conventional synthetic route to *N*-monofluoroalkyl tropanes employs bromo-fluoroalkanes of formula Br-(CH₂)ₙ^{y}F, where n is 2 or 3, and y is 18 or 19. There appears to be a bias in the art towards using such bromo compounds, rather than the iodo analogues. The logic is believed to be that, for simple alkylations (e.g. of sterically unhindered primary amines), there is tendency to form overalkylated products (e.g. quaternary ammonium salts) when using a reactive alkylating agent such as an iodoalkane. Thus, there is a bias toward using bromoalkanes in simple alkylations, as the lower reactivity of the bromoalkane affords improved control over the degree of alkylation.

The problem with such bromo-fluoroalkanes is that they are recognized to be ozone-depleting. They are therefore increasingly less acceptable to use by the relevant Regulatory authorities, and of course would not be used by environmentally-conscious drug or chemical manufacturers. Consequently, their commercial availability is being discontinued, and their continued use is no longer appropriate. There are, however, drug and/or imaging agent products which comprise such *N-*monofluoroalkyl tropanes.

The present invention provides a solution to the continued manufacture of such desirable drug and/or imaging agent products, which avoids the use of such ozone-depleting chemicals. Thus, BFP is a class 1 ozone depleter with an 0.02 - 0.7 ODP (relative to CFC-11 which has a reference value of 1). 3-Fluoro-1-iodo-propane (FIP) has a boiling point of 127 °C [J.Org.Chem., 121, 748-749 (1956)], so is less volatile than BFP (boiling point 98-101 °C). The list of ozone depleting agents in the Montreal Protocol (which deals with reduced use of ozone depleting agents) includes organic molecules, usually alkyl halides, containing fluorine, chlorine or bromine. The most important process (regarding ozone depletion) is the catalytic destruction of ozone by atomic chlorine and bromine. The main source of these elements are CFC compounds (Freons) and halons (bromofluoro compounds). There are, however, no iodine-containing compounds listed - which is an advantage for the iodine-containing alkylating agents of the present invention.

The alkylating agents and methods of the present invention are expected to permit faster *N*-alkylation than with the bromoalkanes of the prior art. In addition, the less prone an amine is to undergo alkylation, the more likely it is that the method of the present invention will provide an improvement.

### Detailed Description of the Invention.

In a first aspect, the present invention provides a method of preparation of an *N-*monofluoroalkyl tropane of Formula (IIIA): 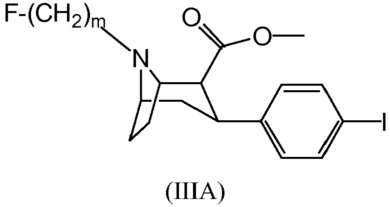 which method comprises:
(i) provision of a precursor which comprises an amine of Formula (III): 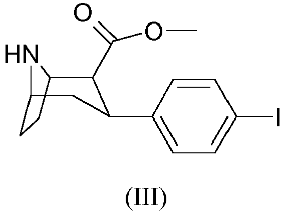
(ii) alkylation of said precursor with an alkylating agent of formula F-(CH₂)₃I in the presence of a base and optionally in the presence of an iodide salt, in a suitable solvent, to give the *N*-monofluoroalkyl tropane of Formula (IIIA),
wherein:
m is 3.

The precursor of Formula (III) is known in the art as *N*-nor-beta-CIT.

The term "base" has its conventional chemical meaning. A preferred such base is an organic base. A preferred organic basis is triethylamine.

By the term "iodide salt" is meant an ionic salt of iodide ion with an alkali metal, preferably sodium or potassium iodide, or a quaternary ammonium iodide (eg. tetrabutylammonium iodide). A preferred such salt is sodium or potassium iodide, most preferably potassium iodide.

Suitable solvents for the alkylation reaction of step (ii) are chosen such that the precursor and alkylating agent are both soluble in the chosen solvent, and that the solvent is stable in the presence of a base. Preferred such solvents include toluene and DMF (dimethylformamide), and related solvents or mixtures thereof. Solvents comprising ketone or ester functional groups are least preferred.

The tropane of Formula (IIIA) and the precursor of Formula (III) are drawn without stereochemistry. The present formulae are intended to encompass all isomers, diastereomers and enantiomers of the chemical structures shown.

### Preferred features.

The precursor of the first aspect is preferably synthetic. The term "synthetic" has the conventional meaning of the term, i.e. man-made as opposed to being isolated from natural sources eg. from the mammalian body. Such compounds have the advantage that their manufacture and impurity profile can be fully controlled.

The alkylating agent is F-(CH₂)₃I.

In the prior art alkylation of *N*-nor-beta-CIT, it is necessary to use potassium iodide to catalyse the alkylation using 1-bromo-3-fluoropropane, together with heating in toluene. Use of 3-fluoro-1-fluoro-propane permits the use of milder reaction conditions, but still without overalkylation. When X is I, it is preferred to carry out the method of the first aspect in the absence of an iodide salt, which simplifies the procedure.

In Formula (IIIA), preferred m values are as specified above, and hence m is most preferably 3. When m = 3, the product of Formula (IIIA) is FP-CIT or Ioflupane. The alkylating agent is F-(CH₂)₃I.

Nor-beta-CIT can be prepared by the method of Neumeyer et al [J.Med.Chem., 37, 1558-1561 (1994)]. It is also commercially available from ABX GmbH, Heinrich-Glaeser-Strasse 10-14 D-01454 Radeberg, Germany.

The alkylating agents of formula F-(CH₂)ₘX, when X =1 are commercially available from Apollo Scientific (Whitefield Rd, Bredbury, Stockport, Cheshire SK6 2QR, USA) and SynQuest Laboratories, Inc. (PO Box 309, Alachua, FL 32616-0309, USA). They can also by prepared from the corresponding triflate F-(CH₂)ₘOTf (where Tf = triflate) by the method of Chi et al [J. Org. Chem., 52, 658-664 (1987)].

In a second aspect, the present invention provides a method of preparation of a trialkyltin radioiodination precursor of Formula IV: 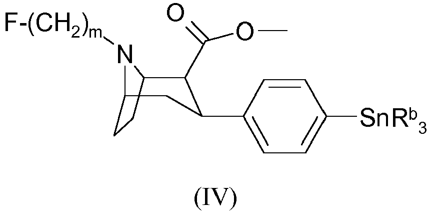 wherein said method comprises:
(i) carrying out the method of the first aspect, to give the *N*-monofluoroalkyl amine product of Formula (IIIA);
(ii) reaction of the compound of Formula (IIIA) from step (i) with Sn₂R^{b}₆ in the presence of a suitable catalyst to give the desired radioiodination precursor of Formula (IV);
where m is as defined in the first aspect, and
each R^{b} is independently C₁₋₄ alkyl.

For the method of the second aspect, m and preferred aspects thereof are as defined in the first aspect.

The organometallic precursors of Formula IV are useful in the preparation of radiopharmaceuticals, as described in the third aspect (below).

Preferred R^{b} groups are methyl and butyl, more preferably methyl. Hexamethylditin is commercially available from Sigma Aldrich. Hexabutylditin is commercially available from Merck Schuchardt & Chemos. The preparation and use of organotin precursors for radioiodination has been described by Bolton [J.Lab.Comp.Radiopharm., 45, 485-528 (2002)] and Ali et al [Synthesis, 423-445 (1996)].

In Formula (IV), m is 3 and R^{b} is preferably methyl, such that the compound of Formula IV is SnFPCT:

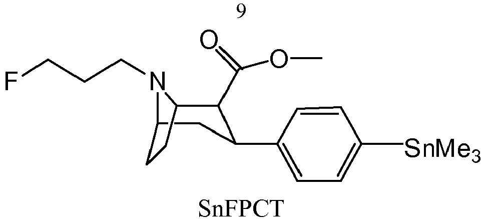

In a third aspect, the present invention provides the use of a compound of Formula (III) as defined in the first aspect as a precursor in the preparation method of the first aspect.

For the use of the third aspect, compounds of Formula (IIIA) or Formula (IV) and preferred aspects thereof are as defined in the first aspect.

In a third aspect, the present invention provides a method of preparation of a radioiodinated tropane of Formula (IIIB): 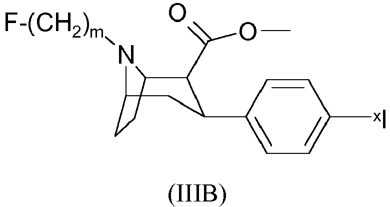 wherein said method comprises:
(a) carrying out the method of the first aspect, to give the *N-*monofluoroalkyl amine product of Formula (IIIA);
(b) carrying out the method of the second aspect, to obtain the radioiodination precursor of Formula IV;
(c) reacting the trialkyltin precursor of Formula IV from step (b) with a supply of radioactive iodide [^{x}I]-iodide, in the presence of a suitable oxidising agent to give the desired product of Formula (IIIB);
where ^{x}I is ¹²⁴I or ¹²³I.

Preferred values of m in the third aspect are as defined in the first aspect. In Formula (IIIB), ^{x}I is preferably ¹²³I, more preferably ^{x}I is ¹²³I and m is 3, such that the radioiodinated compound of Formula (IIIB) is ¹²³I-ioflupane.

The radioiodinated tropane of Formula (IIIB) is preferably provided as a pharmaceutical composition together with a biocompatible carrier medium. By the term "biocompatible carrier medium" is a fluid, especially a liquid, in which the labelled compound is suspended or dissolved, such that the composition is physiologically tolerable, i.e. can be administered to the mammalian body without toxicity or undue discomfort. The biocompatible carrier medium is suitably an injectable carrier liquid such as sterile, pyrogen-free water for injection; an aqueous solution such as saline (which may advantageously be balanced so that the final product for injection is either isotonic or not hypotonic); an aqueous solution of one or more tonicity-adjusting substances (eg. salts of plasma cations with biocompatible counterions), sugars (e.g. glucose or sucrose), sugar alcohols (eg. sorbitol or mannitol), glycols (eg. glycerol), or other non-ionic polyol materials (eg. polyethyleneglycols, propylene glycols and the like). The biocompatible carrier medium may also comprise biocompatible organic solvents such as ethanol. Such organic solvents are useful to solubilise more lipophilic compounds or formulations. Preferably the biocompatible carrier medium is pyrogen-free water for injection, isotonic saline or an aqueous ethanol solution. Such aqueous ethanol solutions may have a range of compositions, but 5-10% ethanol is preferred for the final composition. As indicated above, the pH of the biocompatible carrier medium for intravenous injection is suitably in the range 4.0 to 10.5. For the ¹²³I-labelled radiopharmaceuticals of the present invention, the pH of the biocompatible carrier medium is suitably 4.5 to 8.5, preferably 4.6 to 8.0, most preferably 5.0 to 7.5.

The radiopharmaceutical compositions of the present invention are suitably supplied in a clinical grade syringe or a container which is provided with a seal which is suitable for single or multiple puncturing with a hypodermic needle (e.g. a crimped-on septum seal closure) whilst maintaining sterile integrity. Such containers may contain single doses (a "unit dose") or multiple patient doses. Suitable containers comprise a sealed vessel which permits maintenance of sterile integrity and/or radioactive safety, whilst permitting addition and withdrawal of solutions by syringe. A preferred such container is a septum-sealed vial, wherein the gas-tight closure is crimped on with an overseal (typically of aluminium). Such containers have the additional advantage that the closure can withstand vacuum if desired eg. to change the headspace gas or degas solutions.

When the radiopharmaceutical is supplied in a multiple dose container, preferred such containers comprise a single bulk vial (e.g. of 10 to 30 cm³ volume) which contains enough radiopharmaceutical for multiple patient doses. Unit patient doses can thus be withdrawn into clinical grade syringes at various time intervals during the viable lifetime of the bulk vial preparation to suit the clinical situation.

Radiopharmaceutical syringes designed to contain a single human dose, or "unit dose" and are therefore preferably a disposable or other syringe suitable for clinical use. Such syringes may optionally be provided with a syringe shield to protect the operator from radioactive dose. Suitable such radiopharmaceutical syringe shields are known in the art, and various designs are commercially available, and preferably comprise either lead or tungsten.

The radiopharmaceutical composition may optionally further comprise additional components such as an antimicrobial preservative, pH-adjusting agent or filler. By the term "antimicrobial preservative" is meant an agent which inhibits the growth of potentially harmful micro-organisms such as bacteria, yeasts or moulds. The antimicrobial preservative may also exhibit some bactericidal properties, depending on the dose. The main role of the antimicrobial preservative(s) of the present invention is to inhibit the growth of any such micro-organism in the radiopharmaceutical composition. Suitable antimicrobial preservative(s) include: the parabens, ie. methyl, ethyl, propyl or butyl paraben or mixtures thereof; benzyl alcohol; phenol; cresol; cetrimide and thiomersal. Preferred antimicrobial preservative(s) are the parabens.

The term "pH-adjusting agent" means a compound or mixture of compounds useful to ensure that the pH of the radiopharmaceutical composition is within acceptable limits (approximately pH 4.0 to 8.5) for human or mammalian administration. Suitable such pH-adjusting agents include pharmaceutically acceptable buffers, such as tricine, phosphate buffer or TRIS [ie. *tris*(hydroxymethyl)aminomethane], and pharmaceutically acceptable bases such as sodium carbonate, sodium bicarbonate or mixtures thereof. For ¹²³I-FP-CIT, a preferred buffer is phosphate buffer.

By the term "filler" is meant a pharmaceutically acceptable bulking agent which may facilitate material handling during product production. Suitable fillers include inorganic salts such as sodium chloride, and water soluble sugars or sugar alcohols such as sucrose, maltose, mannitol or trehalose.

The radiopharmaceuticals of the present invention may be prepared under aseptic manufacture conditions to give the desired sterile, pyrogen-free product. The radiopharmaceuticals may also be prepared under non-sterile conditions, followed by terminal sterilisation using e.g. gamma-irradiation; autoclaving; dry heat; membrane filtration (sometimes called sterile filtration); or chemical treatment (e.g. with ethylene oxide).

In a fourth aspect, the present invention provides the use of a compound of formula F-(CH₂)₃I as an alkylating agent in the preparation of either:
(i) the compound of Formula (IIIA) as defined in the first aspect;
(ii) the compound of Formula (IV) as defined in the second aspect;
(iii) the compound of Formula (IIIB) as defined in the third aspect;
wherein m and X are as defined in the first aspect.

In the fourth aspect, the compound of formula F-(CH₂)₃I is preferably used as an alkylating agent in the methods of preparation as described in the first, second and third aspects.

In a fifth aspect, the present invention provides the use of an amine of Formula (III) as defined in the first aspect as a precursor in the method of preparation of the first aspect.

The invention is illustrated by the non-limiting Examples detailed below. Example 1 shows that 3-fluoro-1-iodopropane gives yields at least comparable to 1-bromo-3-fluoropropane in the *N*-alkylation of *N*-nor-β-CIT.

### Example 1: Alternative Synthesis of FP-CIT Using 3-Fluoro-1-iodopropane.

*N*-nor-β-CIT (0.33 mmol, 123 mg) was dissolved in toluene (20 ml/gram, approx. 2 ml). Added to this solution was 3-fluoro-1-iodopropane (0.43 mmol, 81 mg) and triethylamine (0.45 mmol, 45 mg). The reaction mixture was heated to reflux under an inert atmosphere (argon/nitrogen). The reaction was essentially complete (as verified by Thin Layer Chromatography, silica TLC plates eluted with hexane-diethyl ether-triethyl amine) after 6 hours. The solvent was removed by *in vacuo* evaporation, and the crude was treated with diethyl ether (6 ml) and the ether phase was analysed by HPLC (reversed phase C18, gradient acetonitrile-water-phosphate buffer, UV detection at 230 nm). The crude contained 87% area of the desired FP-CIT.

## Claims

1. A method of preparation of an *N*-monofluoroalkyl tropane of Formula (IIIA): 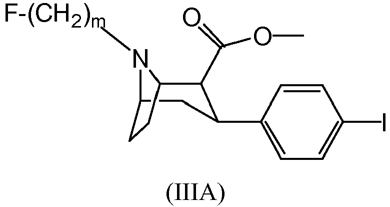 which method comprises:
(i) provision of a precursor which comprises an amine of Formula (III): 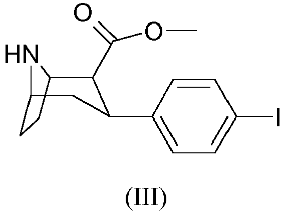
(ii) alkylation of said precursor with an alkylating agent of formula F-(CH₂)₃-I in the presence of a base and optionally in the presence of an iodide salt, in a suitable solvent, to give the *N*-monofluoroalkyl tropane of Formula (IIIA)
wherein:
m is 3.

2. A method of preparation of a trialkyltin radioiodination precursor of Formula IV: 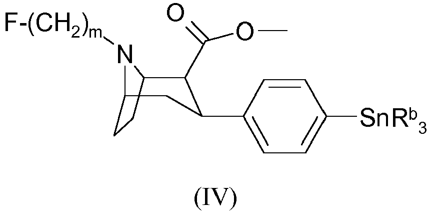 wherein said method comprises:
(i) carrying out the method of claim 1, to give the *N*-monofluoroalkyl amine product of Formula (IIIA);
(ii) reaction of the compound of Formula (IIIA) from step (i) with Sn₂R^{b}₆ in the presence of a suitable catalyst to give the desired radioiodination precursor of Formula (IV);
where m is as defined in claim 1, and
each R^{b} is independently C₁₋₄ alkyl.

3. A method of preparation of a radioiodinated tropane of Formula (IIIB): 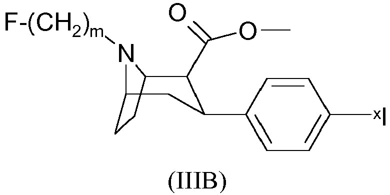 wherein said method comprises:
(a) carrying out the method of claim 1, to give the *N*-monofluoroalkyl amine product of Formula (IIIA);
(b) carrying out the method of claim 2, to obtain the radioiodination precursor of Formula IV;
(c) reacting the trialkyltin precursor of Formula IV from step (b) with a supply of radioactive iodide [^{x}I]-iodide, in the presence of a suitable oxidising agent to give the desired product of Formula (IIIB);
where ^{x}I is ¹²⁴I or ¹²³I, and m is as defined in claim 1.

4. The method of claim 3, where ^{x}I is ¹²³I.

5. The method of claim 4, where the radioiodinated tropane of Formula (IIIB) is provided as a pharmaceutical composition together with a biocompatible carrier medium.

6. The use of a compound of formula F-(CH₂)ₘ I as an alkylating agent in the preparation of either:
(i) the compound of Formula (IIIA) as defined in claim 1;
(ii) the compound of Formula (IV) as defined in claim 2;
(iii) the compound of Formula (IIIB) as defined in claim 3 or claim 4;
where m is as defined in claim 1.

7. The use of claim 6, where the alkylating agent is as defined in claim 1.

8. The use of claim 6 or claim 7, where the method of preparation is as defined in any one of claims 1 to 5.

9. The use of an amine of Formula (III) as defined in Claim 1 as a precursor in the method of preparation of any one of claims 1 to 5.

## Patentansprüche

1. Verfahren zur Herstellung eines *N*-Monofluoralkyltropans der Formel (IIIA): 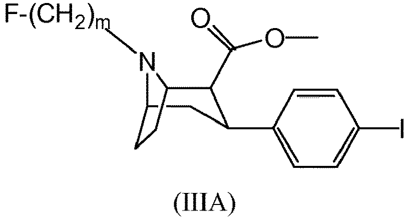 wobei das Verfahren Folgendes umfasst:
(i) Bereitstellen einer Vorstufe, die ein Amin der Formel (III) umfasst: 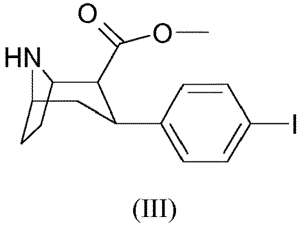
(ii) Alkylieren der Vorstufe mit einem Alkylierungsmittel der Formel F-(CH₂)₃-I in Gegenwart einer Base und wahlweise in Gegenwart eines Iodidsalzes in einem geeigneten Lösemittel, um das *N*-Monofluoralkyltropan der Formel (IIIA) zu ergeben, wobei:
m 3 ist.

2. Verfahren zur Herstellung einer Trialkylzinn-Radioiodierungsvorstufe der Formel IV: 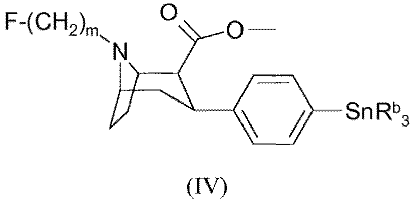 wobei das Verfahren Folgendes umfasst:
(i) Durchführen des Verfahrens nach Anspruch 1, um das *N*-Monofluoralkylamin-Produkt der Formel (IIIA) zu ergeben;
(ii) Umsetzen der Verbindung der Formel (IIIA) aus Schritt (i) mit Sn₂R^{b}₆ in Gegenwart eines geeigneten Katalysators, um die gewünschte Radioiodierungsvorstufe der Formel (IV) zu ergeben;
wobei m wie in Anspruch 1 definiert ist und
jeder R^{b} unabhängig C₁₋₄-Alkyl ist.

3. Verfahren zur Herstellung eines radioiodierten Tropans der Formel (IIIB): 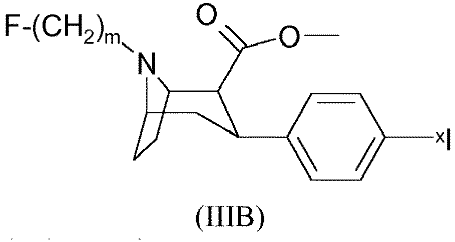 wobei das Verfahren Folgendes umfasst:
(a) Durchführen des Verfahrens nach Anspruch 1, um das *N*-Monofluoralkylamin-Produkt der Formel (IIIA) zu ergeben;
(b) Durchführen des Verfahrens nach Anspruch 2, um die Radioiodierungsvorstufe der Formel IV zu erhalten;
(c) Umsetzen der Trialkylzinn-Vorstufe der Formel IV aus Schritt (b) mit einer Einspeisung von radioaktivem Iodid [^{x}I]-Iodid in Gegenwart eines geeigneten Oxidationsmittels, um das gewünschte Produkt der Formel (IIIB) zu ergeben;
wobei ^{X}I ¹²⁴I oder ¹²³I ist und m wie in Anspruch 1 definiert ist.

4. Verfahren nach Anspruch 3, wobei ^{X}I ¹²³I ist.

5. Verfahren nach Anspruch 4, wobei das radioiodierte Tropan der Formel (IIIB) als pharmazeutische Zusammensetzung gemeinsam mit einem biokompatiblen Trägermedium bereitgestellt wird.

6. Verwendung einer Verbindung der Formel F-(CH₂)ₘ-I als Alkylierungsmittel bei der Herstellung von entweder:
(i) der Verbindung der Formel (IIIA) wie in Anspruch 1 definiert;
(ii) der Verbindung der Formel (IV) wie in Anspruch 2 definiert;
(iii) der Verbindung der Formel (IIIB) wie in Anspruch 3 oder Anspruch 4 definiert;
wobei m wie in Anspruch 1 definiert ist.

7. Verwendung nach Anspruch 6, wobei das Alkylierungsmittel wie in Anspruch 1 definiert ist.

8. Verwendung nach Anspruch 6 oder Anspruch 7, wobei das Verfahren zur Herstellung wie in einem der Ansprüche 1 bis 5 definiert ist.

9. Verwendung eines Amins der Formel (III) wie in Anspruch 1 definiert als Vorstufe bei dem Verfahren zur Herstellung nach einem der Ansprüche 1 bis 5.

## Revendications

1. Procédé de préparation d'un *N*-monofluoroalkyl tropane de Formule (IIIA) : 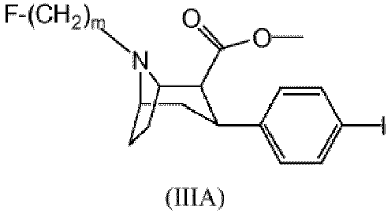 lequel procédé comprend :
(i) la fourniture d'un précurseur qui comprend une amine de Formule (III) : 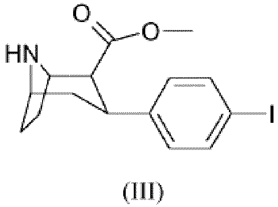
(ii) l'alkylation dudit précurseur avec un agent d'alkylation de formule F-(CH₂)₃-I en présence d'une base et facultativement en présence d'un sel d'iodure, dans un solvant approprié, pour donner le *N*-monofluoroalkyl tropane de Formule (IIIA), dans lequel :
m est 3.

2. Procédé de préparation d'un précurseur de radio-iodation à base de trialkylétain de Formule IV : 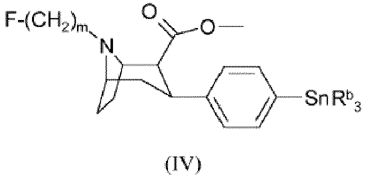 dans lequel ledit procédé comprend :
(i) la mise en œuvre du procédé selon la revendication 1, pour donner le produit *N*-monofluoroalkyl amine de Formule (IIIA) ;
(ii) la réaction du composé de Formule (IIIA) provenant de l'étape (i) avec Sn₂R^{b}₆ en présence d'un catalyseur approprié pour donner le précurseur de radio-iodation souhaité de Formule (IV) ;
m étant tel que défini dans la revendication 1, et
chaque R^{b} est indépendamment un alkyle en C₁ à C₄.

3. Procédé de préparation d'un tropane radio-iodé de Formule (IIIB) : 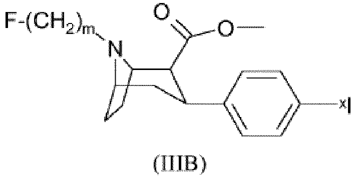 dans lequel ledit procédé comprend :
(a) la mise en œuvre du procédé selon la revendication 1, pour donner le produit *N*-monofluoroalkyl amine de Formule (IIIA) ;
(b) la mise en œuvre du procédé selon la revendication 2, pour obtenir le précurseur de radio-iodation de Formule IV ;
(c) la réaction du précurseur à base de trialkylétain de Formule IV provenant de l'étape (b) avec une alimentation iodure radioactif [^{x}I]-iodure, en présence d'un agent oxydant approprié pour donner le produit souhaité de Formule (IIIB) ;
^{x}I étant ¹²⁴I ou ¹²³I, et m étant tel que défini dans la revendication 1.

4. Procédé selon la revendication 3, dans lequel ^{x}I est ¹²³I.

5. Procédé selon la revendication 4, dans lequel le tropane radio-iodé de Formule (IIIB) est fourni sous la forme d'une composition pharmaceutique conjointement avec un milieu de support biocompatible.

6. Utilisation d'un composé de formule F-(CH₂)ₘI comme agent d'alkylation dans la préparation :
(i) du composé de formule (IIIA) tel que défini dans la revendication 1 ;
(ii) du composé de formule (IV) tel que défini dans la revendication 2 ;
(iii) du composé de formule (IIIB) tel que défini dans la revendication 3 ou la revendication 4 ;
m étant tel que défini dans la revendication 1.

7. Utilisation selon la revendication 6, dans lequel l'agent d'alkylation est tel que défini dans la revendication 1.

8. Utilisation selon la revendication 6 ou la revendication 7, dans laquelle le procédé de préparation est tel que défini dans l'une quelconque des revendications 1 à 5.

9. Utilisation d'une amine de Formule (III) telle que définie dans la revendication 1 comme précurseur dans le procédé de préparation selon l'une quelconque des revendications 1 à 5.
